# EUROPEAN PATENT SPECIFICATION

(11) **EP 4 247 258 B1**
(45) Date of publication and mention of the grant of the patent: **28.01.2026**
(21) Application number: 21816618.9
(22) Date of filing: 09.11.2021
(51) Int. Cl.: A61M 25/00, A61B 5/15, A61M 25/06, A61B 5/154

(54) **CATHETER SYSTEM**
KATHETERSYSTEM
SYSTÈME DE CATHÉTER

(30) Priority: 18.11.2020 CN 202011289322
(43) Date of publication of application: 27.09.2023
(73) Proprietor: Becton, Dickinson and Company, Franklin Lakes, NJ 07417 (US)
(72) Inventor: XUE, Yueqiang, Shanghai 201203 (CN); YAN, Bo, Shanghai 200040 (CN); BURKHOLZ, Jonathan Karl, Salt Lake City, Utah 84108 (US); NATESAN, Mohankumar, Bukit Batok 659246 (SG); CHENG, Kiat Jin, Bishan 570125 (SG)
(74) Representative: dompatent
(86) International application number: PCT/US2021/058606
(87) International publication number: WO 2022/108786

(56) References cited:
- WO-A1-2011/146764
- WO-A1-2017/029361
- US-A- 4 444 203
- US-A1- 2019 314 614
- US-A1- 2020 129 738

## Description

### TECHNICAL FIELD

The present invention relates to a catheter system according to the preamble of claim 1. Such a catheter system is known from US 2020129738 A1.

### BACKGROUND

Intravenous catheters are commonly used for a variety of infusion therapies. For example, intravenous catheters may be used for infusing fluids, such as normal saline solution, various medicaments, and total parenteral nutrition, into a patient. Intravenous catheters may also be used for withdrawing blood from the patient.

Common types of intravenous catheter are peripheral IV catheters ("PIVCs"), peripherally inserted central catheters ("PICCs"), and midline catheters. Intravenous catheters may include "over-the needle" catheters, which may be mounted over a needle having a sharp distal tip. The sharp distal tip may be used to pierce skin and the vasculature of the patient. Insertion of the intravenous catheter into the vasculature may follow the piercing of the vasculature by the needle. The needle and the intravenous catheter are generally inserted at a shallow angle through the skin into the vasculature of the patient with a bevel of the needle facing up and away from the skin of the patient.

In order to verify proper placement of the introducer needle and/or the intravenous catheter in the vasculature, a user generally confirms that there is flashback of blood, which may be visible to the user. In some instances, the introducer needle may include a notch disposed towards a distal end of the introducer needle, and in response to the distal tip of the introducer needle being positioned within the vasculature, blood may flow proximally through a needle lumen, exit the needle lumen through the notch, and then travel proximally between an outer surface of the introducer needle and an inner surface of the intravenous catheter.

Accordingly, where the intravenous catheter is at least partially transparent, the user may visualize a small amount of blood "flashback" and thereby confirm placement of the intravenous catheter within the vasculature. Presence of a vasculature entrance indicator, such as flashback, may facilitate successful placement of intravenous catheters. Once placement of the introducer needle within the vasculature has been confirmed, the user may temporarily occlude flow in the vasculature and withdraw the introducer needle, leaving the intravenous catheter in place for future blood withdrawal and/or fluid infusion.

The intravenous catheter may extend distally from a catheter adapter, which may include a side port. The side port may be coupled to an extension set, which may be coupled to a blood collection device. When the intravenous catheter is inserted into the vasculature of the patient, blood may flow from the patient through the catheter and into the extension set and blood collection device. Collecting blood this way poses several challenges, including contamination of a blood sample with priming fluid, blood residue at a proximal end of the catheter adapter even after flushing, and an increased risk of blood exposure.

US2020129738A1 discloses a catheter system including a catheter adapter, a catheter extending distally from the catheter adapter, an introducer needle extending through the catheter, a flash chamber in fluid communication with a needle lumen of the introducer needle, and a seal element. The introducer needle may include a wall defining the needle lumen, a first notch formed through the wall, and a second notch formed through the wall. The seal element may be proximal to the first notch and the second notch and within the catheter between an outer surface of the introducer needle and an inner surface of the catheter. The seal element may prevent priming fluid from travelling distal to the seal element. The seal element in conjunction with the first notch, the second notch, and the flash chamber may facilitate pressure-driven blood flow into the catheter for detection of flashback and transfixing when priming occurs prior to insertion into vasculature.

The subject matter claimed herein is not limited to embodiments that solve any disadvantages or that operate only in environments such as those described above. Rather, this background is only provided to illustrate one example technology area where some implementations described herein may be practiced.

### SUMMARY

The present disclosure generally relates to blood collection devices, systems, and non claimed methods. According to the present invention there is provided a catheter system comprising the features of claim 1. Preferred embodiments of the present invention are defined in claims 2-9.

In some embodiments, the blood collection tube may include a VACUTAINER tube, which may include a hermetic seal at an open end and a vacuum. The vacuum in the VACUTAINER tube may cause the blood sample to be drawn, through at least a portion of the catheter and then the introducer needle. In some embodiments, from the introducer needle, the blood may flow through one or more of the following: the needle hub, the extension tube, the cannula hub, and the cannula.

In some embodiments, the catheter system may include a peripheral intravenous catheter ("PIVC") system. In some embodiments, the catheter system may include a peripherally inserted central catheter ("PICC") system or a midline catheter system.

In some embodiments, the catheter adapter may include a side port. In some embodiments, the catheter system may include another extension tube. In some embodiments, the other extension tube may include a distal end and a proximal end. In some embodiments, the distal end of the other extension tube may be integrated with the side port. In some embodiments, the proximal end of the extension tube may be integrated with an adapter, such as a Y-adapter or another suitable adapter.

In some embodiments, the catheter may include a flashback notch. In some embodiments, flashback may flow from an outside of the catheter through the flashback notch to a space in between the introducer needle and the catheter. In some embodiments, flashback may be visible through the catheter such that a clinician may determine that the catheter has entered a vasculature of a patient. In some instances, the flashback notch of the catheter may more accurately indicate to the clinician presence of the catheter within the vasculature, compared to a flashback notch in the introducer needle. Thus, the flashback notch of the catheter may increase first-stick success. In some embodiments, the catheter may be translucent. In some embodiments, the catheter system may be vented at one or more locations, which may facilitate proximal flow of flashback. For example, a vent plug or air venting cap may be coupled to the adapter.

In some embodiments, the catheter system may include a needle shield, which may be at least partially disposed within the catheter adapter. In some embodiments, the needle shield may include a V-clip or any other suitable needle shield. In some embodiments, in response to removing the introducer needle from the catheter adapter, the needle shield may cover the sharp distal tip of the introducer needle.

According to the present invention, the proximal end of the catheter adapter includes an opening. According to the present invention, the catheter assembly includes a clip disposed within the catheter adapter distal to the opening. In some embodiments, the clip may include a first arm, which may include a first hole. In some embodiments, the clip may include a second arm, which may include a second hole. In some embodiments, in response to the introducer needle being in an insertion position ready for insertion into the patient, the introducer needle may extend through the first hole and the second hole and the arms may be biased inwardly. In some embodiments, in response to the introducer needle being proximally withdrawn from the first hole and the second hole, the first arm and the second arm may move resiliently outward such that the first hole and the second hole move away from each other and the clip blocks the opening.

In some embodiments, the first arm and the second arm may be joined at a first bend. In some embodiments, the first arm may include a second bend and the second arm may include a third bend. In some embodiments, the first hole may be disposed inward to the second bend. In some embodiments, the second hole may be disposed inward to the third bend. In some embodiments, the first arm may include a first end. In some embodiments, the second arm may include a second end. In some embodiments, in response to the introducer needle being proximally withdrawn from the first hole and the second hole, the first end and the second end may overlap.

In some embodiments, a non claimed method of collecting blood from a patient may include inserting the catheter system into the vasculature of the patient. In some embodiments, the catheter system may include a wedge, which may be constructed of metal. In some embodiments, the wedge may secure the catheter within the catheter adapter. In some embodiments, the method may include separating the needle hub from the catheter adapter such that a distal end of the introducer needle is disposed within the wedge. In some embodiments, the needle hub may be separated from the catheter adapter by moving the needle hub proximally with respect to the catheter adapter.

In some embodiments, the method may include collecting blood within the blood collection tube when the distal end of the introducer needle is disposed within the wedge. In some embodiments, collecting blood within the blood collection tube when the distal end of the introducer needle is disposed within the wedge may include pushing the blood collection tube distally against the elastomeric sheath such that the cannula pierces the elastomeric sheath and a fluid pathway is formed between the cannula and the blood collection tube.

In some embodiments, the method may include priming or flushing the catheter system with a solution prior to collecting blood within the blood collection tube and/or insertion of the catheter system into the vasculature of the patient. In some embodiments, the solution may include a priming solution or a flushing solution. In some embodiments, the solution may include saline. In some embodiments, a syringe or other device configured to flush or prime the catheter system may be filled with the solution and coupled to the adapter. In some embodiments, the solution may flow distally through the other extension tube and the catheter in response to activation of the syringe or other device. In some embodiments, a "waste" sample that is contaminated with solution does not need to be collected prior to collecting blood within the blood collection tube because the solution does not contact the lumen of the introducer needle (due to the lack of a flashback notch).

In some embodiments, the method may include removing the introducer needle from the catheter adapter. In some embodiments, in response to removing the introducer needle from the catheter adapter, the needle shield may cover the sharp distal tip, which may reduce a risk of needle stick. In some embodiments, the method may include flushing the catheter via the extension tube after collecting blood within the blood collection tube.

It is to be understood that both the foregoing general description and the following detailed description are examples and explanatory and are not restrictive of the invention, as claimed. It should be understood that the various embodiments are not limited to the arrangements and instrumentality shown in the drawings. It should also be understood that the embodiments may be combined, or that other embodiments may be utilized and that structural changes, unless so claimed, may be made without departing from the scope of the various embodiments of the present invention. The following detailed description is, therefore, not to be taken in a limiting sense.

### BRIEF DESCRIPTION OF THE SEVERAL VIEWS OF THE DRAWINGS

Example embodiments will be described and explained with additional specificity and detail through the use of the accompanying drawings in which:
Figure 1A is an upper perspective view of a prior art catheter system;
Figure 1B is an exploded view of the prior art catheter system of Figure 1A;
Figure 2A is an upper perspective view of an example needle assembly, according to some embodiments;
Figure 2B is a cross-sectional view of the needle assembly of Figure 2A, according to some embodiments;
Figure 3A is an upper perspective view of an example catheter system in an insertion position ready for insertion into a patient, according to some embodiments;
Figure 3B is an upper perspective view of the catheter system of Figure 3A coupled to a blood collection tube, according to some embodiments;
Figure 3C is a cross-sectional view of an example catheter assembly, according to some embodiments;
Figure 4A is an upper perspective view of another needle assembly, according to some embodiments;
Figure 4B is a cross-sectional view of the needle assembly of Figure 4A, according to some embodiments;
Figure 4C is an upper perspective view of another catheter assembly, according to some embodiments;
Figure 4D is an upper perspective view of another catheter system, according to some embodiments;
Figure 4E is a top view of an example distal end of the catheter system of Figure 4D, according to some embodiments;
Figure 4F is an upper perspective view of an example flushing or priming device being inserted into the catheter system of Figure 4D, according to some embodiments;
Figure 5A is a cross-sectional view of an example catheter adapter, illustrating an example clip in a first position, according to some embodiments; and
Figure 5B is a cross-sectional view of the catheter adapter of Figure 5A, illustrating the clip in a second position, according to some embodiments.

### DESCRIPTION OF EMBODIMENTS

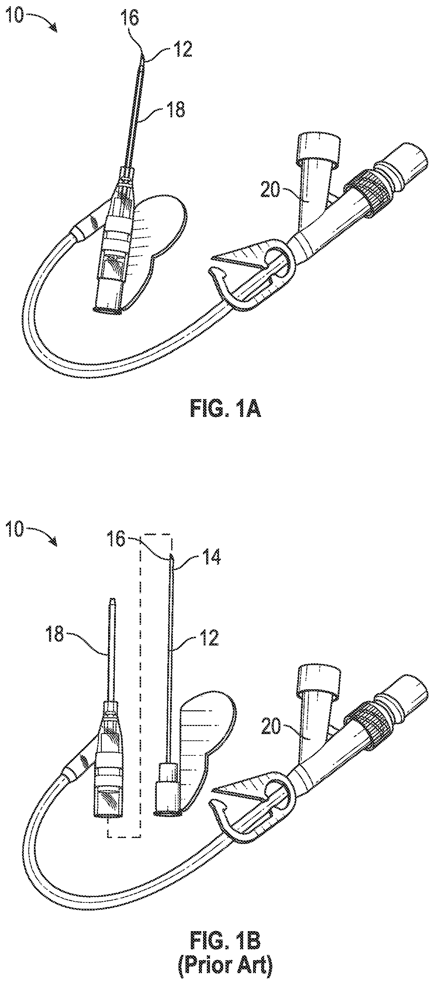

Referring now to Figures 1A-1B, a prior art catheter system 10 is illustrated. In some embodiments, the introducer needle 12 may include a flashback notch 14. In some embodiments, flashback may flow into a distal end 16 of the introducer needle 12 and out the flashback notch 14 to a space between the introducer needle 12 and a catheter 18. The flashback may indicate to a clinician that the introducer needle 12 is positioned within a vasculature of a patient.

Before blood collection from the patient using the prior art catheter system 10, the prior art catheter system 10 may be flushed or primed with a solution. Thus, when a blood sample is collected, due to the presence of the flashback notch 14 on the introducer needle 12, the blood sample may be contaminated with the solution.

Following blood collection, a needleless connector or PRN adapter (not illustrated) connected to an adapter 20 of the prior art catheter system 10 may need to be removed, which may increase a risk of bacterial contamination of the prior art catheter system 10. Further, the needleless connector or PRN adapter connected to an adapter 20 may need to be removed after blood collection and/or flushing of the prior art catheter system 10, which may result in extra work for the clinician.

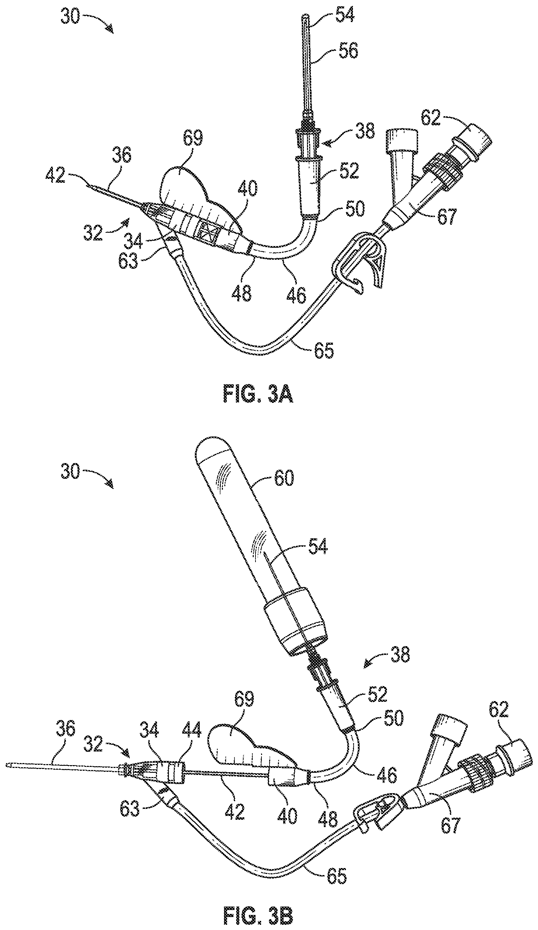

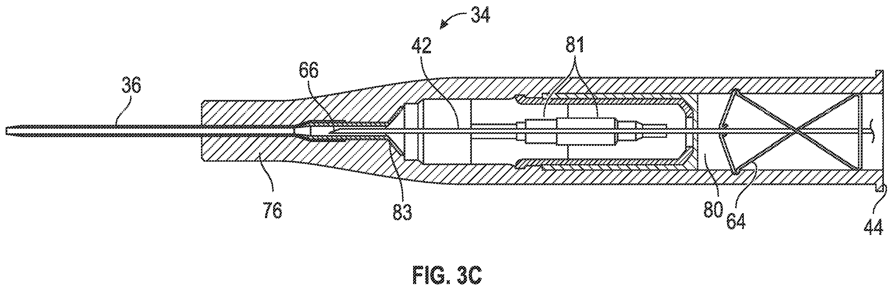

Referring now to Figures 3A-3B, in some embodiments, a catheter system 30 may include a catheter assembly 32, which may include a catheter adapter 34 and a catheter 36 extending distally from the catheter adapter 34. In some embodiments, the catheter system 30 may include a needle assembly 38, which may include a needle hub 40 and an introducer needle 42 extending distally from the needle hub 40.

In some embodiments, the introducer needle 42 may extend through the catheter 36 when the catheter system 30 is in the insertion position, as illustrated, for example, in Figure 3A. In some embodiments, the needle hub 40 may be proximate and proximal to the catheter adapter 34. In some embodiments, the needle hub 40 may be coupled to the catheter adapter 34. In some embodiments, the needle hub 40 may not be coupled to the catheter adapter 34, which may facilitate retraction of the introducer needle 42 and the needle hub 40 in a proximal direction. In these and other embodiments, the needle hub 40 may not extend distal to a proximal end 44 of the catheter adapter 34.

In some embodiments, the needle assembly 38 may include an extension tube 46, which may include a distal end 48 integrated with the needle hub 40 and a proximal end 50 integrated with a cannula hub 52. In some embodiments, a cannula 54 may extend proximally from the cannula hub 52. In some embodiments, an elastomeric sheath 56 may be coupled to the cannula hub 52, and a proximal end 58 of the cannula 54 may be enveloped within the elastomeric sheath 56. In some embodiments, in response to a blood collection tube 60 pushing the elastomeric sheath 56 distally, the cannula 54 may pierce the elastomeric sheath 56, and the cannula 54 may insert into the blood collection tube 60.

Figure 3B illustrates the needle assembly 38 partially withdrawn from the catheter adapter 34, which may facilitate blood collection. In some embodiments, the blood collection tube 60 may include a VACUTAINER tube, which may include a hermetic seal at an open end and a vacuum. The vacuum in the VACUTAINER tube may cause the blood sample to be drawn, through at least a portion of the catheter 36 and then the introducer needle 42. In some embodiments, from the introducer needle 42, the blood may flow through one or more of the following: the needle hub 40, the extension tube 46, the cannula hub 52, and the cannula 54.

In some embodiments, the catheter system 30 may include a peripheral intravenous catheter ("PIVC") system. In some embodiments, the catheter system 30 may include a peripherally inserted central catheter ("PICC") system or a midline catheter system.

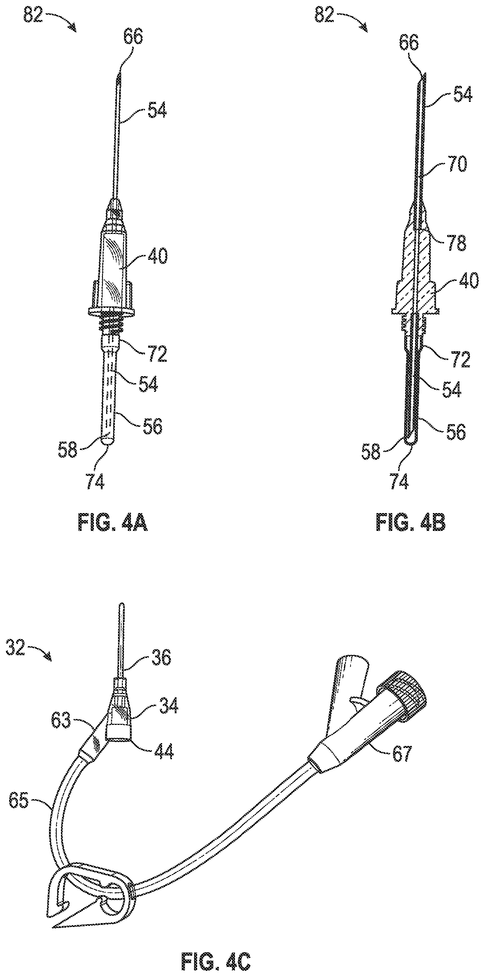

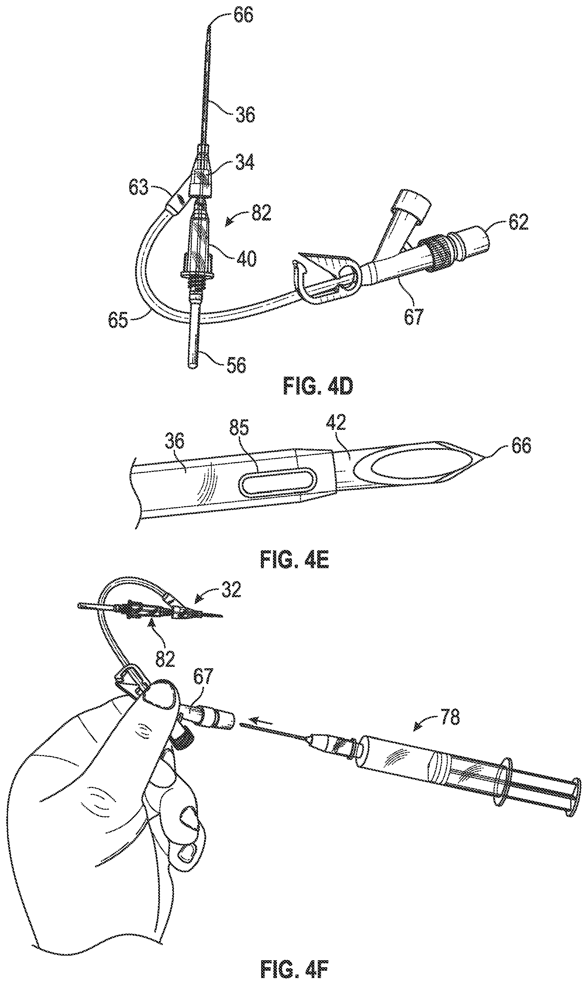

In some embodiments, the catheter 18 may include a flashback notch. In some embodiments, the flashback notch may be illustrated in Figure 4E. In some embodiments, flashback may flow from an outside of the catheter 18 through the flashback notch to a space in between the introducer needle 42 and the catheter 18. In some embodiments, flashback may be visible through the catheter 18 such that a clinician may determine that the catheter 18 has entered a vasculature of a patient. In some instances, the flashback notch of the catheter 18 may more accurately indicate to the clinician presence of the catheter 18 within the vasculature, compared to a flashback notch in the introducer needle 42. Thus, the flashback notch of the catheter 18 may increase first-stick success. In some embodiments, the catheter 18 may be translucent. In some embodiments, the catheter system 30 may be vented at one or more locations, which may facilitate proximal flow of flashback. For example, a vent plug 62 or air venting cap may be coupled to the adapter.

In some embodiments, the catheter adapter 34 may include a side port 63. In some embodiments, the catheter system 30 may include another extension tube 65. In some embodiments, the other extension tube 65 may include a distal end and a proximal end. In some embodiments, the distal end of the other extension tube 65 may be integrated with the side port 63. In some embodiments, the proximal end of the extension tube 65 may be integrated with an adapter 67, such as a Y-adapter or another suitable adapter. In some embodiments, the catheter adapter 34 may include a paddle grip 69, which may be generally planar. In some embodiments, the paddle grip 69 may extend distally from the needle hub 40.

Referring now to Figures 3A-3C, the catheter adapter 34 is illustrated with the needle assembly 38 partially withdrawn, according to some embodiment. In some embodiments, the catheter system 30 may include a needle shield 64, which may be at least partially disposed within the catheter adapter 34. In some embodiments, the needle shield 64 may include a V-clip or any other suitable needle shield. In some embodiments, in response to removing the introducer needle 42 from the catheter adapter 34, the needle shield 64 may cover a sharp distal tip 66 of the introducer needle 42.

In some embodiments, the catheter adapter 34 may include a distal end 76, the proximal end 44, and a lumen 80 extending through the distal end 76 and the proximal end 44 of the catheter adapter 34. In some embodiments, the catheter adapter 34 may include a septum 81, which may be short in length in response to a lack of the flashback notch in the introducer needle 42 and may have less drag force.

In some embodiments, a non claimed method of collecting blood from the patient may include inserting the catheter system 30 into the vasculature of the patient. In some embodiments, the catheter system 30 may include a wedge 83, which may be constructed of metal. In some embodiments, the wedge 83 may secure the catheter 36 within the catheter adapter 34. In some embodiments, the method may include separating the needle hub 40 from the catheter adapter 34 such that the sharp distal tip 66 of the introducer needle 42 is disposed within the wedge 83. In some embodiments, the sharp distal tip 66 of the introducer needle 42 may be disposed within the wedge 83 during blood collection such that the sharp distal tip 66 does not injure the catheter assembly 32 and/or a majority if not all of blood flowing proximally through the catheter 36 enters the introducer needle 42. In some embodiments, an outer diameter of the introducer needle 42 may be equal to or slightly less than an inner diameter of a neck of the wedge 83, which may be proximate a tapered mouth of the wedge 83. The mouth may include a larger inner diameter than the neck. In some embodiments, the needle hub 40 may be separated from the catheter adapter 34 by moving the needle hub 40 proximally with respect to the catheter adapter 34.

In some embodiments, the method may include collecting blood within the blood collection tube 60 when the distal end of the introducer needle 42 is disposed within the wedge 83. In some embodiments, collecting blood within the blood collection tube 60 when the distal end of the introducer needle 42 is disposed within the wedge 83 may include pushing the blood collection tube 60 distally against the elastomeric sheath 56 such that the cannula 54 pierces the elastomeric sheath 56 and a fluid pathway is formed between the cannula 54 and the blood collection tube 60.

In some embodiments, the method may include priming or flushing the catheter system 30 with a solution prior to collecting blood within the blood collection tube 60 and/or insertion of the catheter system 30 into the vasculature of the patient. In some embodiments, the solution may include saline. In some embodiments, a syringe or other device configured to flush or prime the catheter system 30 may be filled with the solution and coupled to the adapter 67. In some embodiments, the solution may flow distally through the other extension tube 65 and the catheter 36 in response to activation of the syringe or other device. In some embodiments, a "waste" sample that is contaminated with solution does not need to be collected prior to collecting blood within the blood collection tube 60 because the solution does not contact the lumen 70 of the introducer needle 42 (due to the lack of a flashback notch in the introducer needle 42).

In some embodiments, the method may include removing the introducer needle 42 from the catheter adapter 34. In some embodiments, in response to removing the introducer needle 12 from the catheter adapter 34, the needle shield 64 may cover the sharp distal tip 66, which may reduce a risk of needle stick. In some embodiments, the method may include flushing the catheter 36 via the other extension tube 65 after collecting blood within the blood collection tube 60.

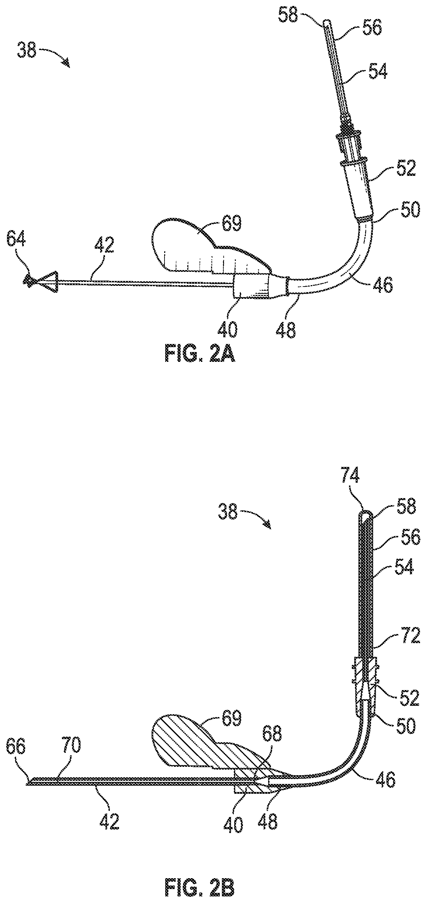

Referring now to Figures 2A-2B, the needle assembly 38 is illustrated, according to some embodiments. In some embodiments, the introducer needle 42 may include a proximal end 68, the sharp distal tip 66, and a lumen 70 disposed between the proximal end 68 and the sharp distal tip 66. In some embodiments, the elastomeric sheath 56 may include an open distal end 72 and a closed proximal end 74. In some embodiments, the open distal end 72 may be mounted on the cannula hub 52.

Referring now to Figures 4A-4B, another needle assembly 82 is illustrated, according to some embodiments. In some embodiments, the needle assembly 82 may be used with the catheter system 30 or another suitable catheter system. In some embodiments, the needle assembly 82 may include one or more features of the needle assembly 38 discussed with respect to Figures 2-3 and/or may operate similar to the needle assembly 38 in one or more respects. In some embodiments, the cannula 54 may extend proximally from the needle hub 40. In some embodiments, the elastomeric sheath 56 may be coupled to the cannula hub 52, and the proximal end 58 of the cannula 54 may be enveloped within the elastomeric sheath 56.

Referring now to Figure 4C, the catheter assembly 32 is illustrated, according to some embodiments. Referring now to Figure 4D, the needle assembly 82 may be inserted into the catheter assembly 32, as illustrated, for example, in Figure 4D. Referring now to Figure 4E, in some embodiments, the catheter 36 of the catheter assembly 32 may include the flashback notch 85. Referring now to Figure 4F, when the needle assembly 82 is coupled to the catheter assembly 32, a flushing or priming device 78 may be being inserted into the catheter assembly 32. In some embodiments, the flushing or priming device 78 may be coupled to a needleless connector or PRN, which may be coupled to the adapter 67.

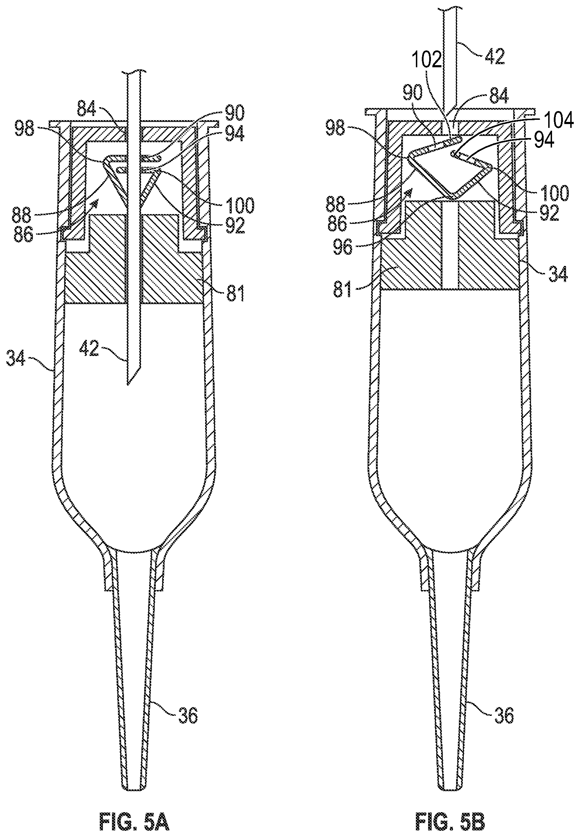

Referring now to Figures 5A-5B, in some embodiments, the proximal end 44 of the catheter adapter 34 may include an opening 84. In some embodiments, the opening 84 may be disposed in an insert of the catheter adapter 34. In some embodiments, the catheter adapter 34 may include a clip 86 disposed within the catheter adapter 34 distal to the opening 84. In some embodiments, the clip 86 may include a first arm 88, which may include a first hole 90. In some embodiments, the clip 86 may include a second arm 92, which may include a second hole 94. In some embodiments, in response to the introducer needle 42 being in an insertion position ready for insertion into the patient, the introducer needle 42 may extend through the first hole 90 and the second hole 94, and the first arm 88 and the second arm 92 may be biased inwardly. In some embodiments, in response to the introducer needle 42 being proximally withdrawn from the first hole 90 and the second hole 94, the first arm 88 and the second arm 92 may move resiliently outward such that the first hole 90 and the second hole 94 move away from each other and the clip 86 may block the opening 84.

In some embodiments, the first arm 88 and the second arm 92 may be joined at a first bend 96. In some embodiments, the first arm 88 may include a second bend 98 and the second arm 92 may include a third bend 100. In some embodiments, the first hole 90 may be disposed inward to the second bend 98. In some embodiments, the second hole 94 may be disposed inward to the third bend 100. In some embodiments, the first arm 88 may include a first end 102. In some embodiments, the second arm 92 may include a second end 104. In some embodiments, in response to the introducer needle 42 being proximally withdrawn from the first hole 90 and the second hole 94, the first end 102 and the second end 104 may overlap.

## Claims

1. A catheter system (30), comprising:
a catheter assembly (32), comprising:
a catheter adapter (34), comprising a distal end (76), a proximal end (44), and a lumen (80) extending through the distal end and the proximal end; and
a catheter (36) extending distally from the distal end of the catheter adapter; and
a needle assembly (38), comprising:
a needle hub (40) proximate and proximal to the catheter adapter;
an introducer needle (42) extending distally from the needle hub;
a cannula (54) extending proximally from the needle hub; and
**CHARACTERIZED IN THAT**
an elastomeric sheath (56) is coupled to the needle hub, wherein a proximal end of
the cannula is enveloped within the elastomeric sheath, wherein the elastomeric sheath comprises an open distal end (72) and a closed proximal end (74),
the catheter assembly further comprises a clip (86) disposed distal to an opening (84) of the proximal end of the catheter adapter, wherein the clip comprises a first hole (90), wherein the introducer needle extends through the first hole when the introducer needle is in an insertion position.

2. The catheter system of claim 1, wherein the catheter comprises a flashback notch (85).

3. The catheter system of claim 1, wherein the cannula is configured to insert into a blood collection tube (60), wherein in response to the blood collection tube pushing the elastomeric sheath distally, the cannula pierces the elastomeric sheath.

4. The catheter system of claim 1, wherein the catheter adapter comprises a side port (63), wherein the catheter system further comprises another extension tube (65), wherein the other extension tube comprises a distal end and a proximal end, wherein the distal end of the other extension tube is integrated with the side port, wherein the proximal end of the other extension tube is integrated with an adapter (67).

5. The catheter system of claim 1, further comprising a needle shield (64) at least partially disposed within the catheter adapter, wherein in response to removing the introducer needle from the catheter adapter, the needle shield covers a sharp distal tip (66) of the introducer needle.

6. The catheter system of claim 1, wherein the clip comprises a first arm (88) having the first hole (90) and a second arm (92) having a second hole (94), wherein in response to the introducer needle being in the insertion position, the introducer needle extends through the first hole and the second hole and the arms are biased inwardly, wherein in response to the introducer needle being proximally withdrawn from the first hole and the second hole, the first arm and the second arm move resiliently outward such that the first hole and the second hole move away from each other and the clip blocks the opening.

7. The catheter system of claim 6, wherein the first arm and the second arm are joined at a first bend (96).

8. The catheter system of claim 7, wherein the first arm comprises a second bend (98) and the second arm comprises a third bend (100), wherein the first hole is disposed inward to the second bend, wherein the second hole is disposed inward to the third bend.

9. The catheter system of claim 8, wherein the first arm comprises a first end (102), wherein the second arm comprises a second end (104), wherein in response to the introducer needle being proximally withdrawn from the first hole and the second hole, the first end and the second end overlap.

## Patentansprüche

1. Kathetersystem (30), welches aufweist:
eine Katheteranordnung (32), welche aufweist:
einen Katheteradapter (34), der ein distales Ende (76), ein proximales Ende (44), und
ein sich durch das distale Ende und das proximale Ende erstreckendes Lumen (80) aufweist; und
einen Katheter (36), der sich distal von dem distalen Ende des Katheteradapters aus erstreckt; und
eine Nadelanordnung (38), welche aufweist:
einen Nadelansatz (40), der sich in der Nähe und proximal des Katheteradapters befindet;
eine Einführnadel (42), die sich distal von dem Nadelansatz erstreckt;
eine Kanüle (54), die sich proximal vom Nadelansatz erstreckt; und **dadurch gekennzeichnet, dass**
eine elastomere Hülle (56) mit dem Nadelansatz verbunden ist, wobei ein proximales Ende der Kanüle von der elastomeren Hülle umschlossen ist, wobei die elastomere Hülle ein offenes distales Ende (72) und ein geschlossenes proximales Ende (74) aufweist,
die Katheteranordnung ferner eine Klemme (86) umfasst, die distal zu einer Öffnung (84) des proximalen Endes des Katheteradapters angeordnet ist, wobei die Klemme ein erstes Loch (90) umfasst, wobei sich die Einführnadel durch das erste Loch erstreckt, wenn sich die Einführnadel in einer Einführposition befindet.

2. Kathetersystem nach Anspruch 1, bei welchem der Katheteradapter eine Flashback-Kerbe (85) aufweist.

3. Kathetersystem nach Anspruch 1, bei welchem die Kanüle so ausgebildet ist, dass sie in ein Blutsammelröhrchen (60) eingeführt werden kann, wobei die Kanüle als Reaktion darauf, dass das Blutsammelröhrchen die elastomere Hülle nach distal drückt, die elastomere Hülle durchsticht.

4. Kathetersystem nach Anspruch 1, bei welchem der Katheteradapter einen Seitenanschluss (63) umfasst, wobei das Kathetersystem ferner einen weiteren Verlängerungsschlauch (65) umfasst, wobei der weitere Verlängerungsschlauch ein distales Ende und ein proximales Ende umfasst, wobei das distale Ende des weiteren Verlängerungsschlauchs mit dem Seitenanschluss integriert ist, wobei das proximale Ende des weiteren Verlängerungsschlauchs mit einem Adapter (67) integriert ist.

5. Kathetersystem nach Anspruch 1, ferner umfassend einen Nadelschutz (64), der zumindest teilweise innerhalb des Katheteradapters angeordnet ist, wobei der Nadelschutz als Reaktion auf das Entfernen der Einführnadel aus dem Katheteradapter eine scharfe distale Spitze (66) der Einführnadel abdeckt.

6. Kathetersystem nach Anspruch 1, bei welchem die Klemme einen ersten Arm (88) mit dem ersten Loch (90) und einen zweiten Arm (92) mit einem zweiten Loch (94) umfasst, wobei sich die Einführnadel als Reaktion darauf, dass sich die Einführnadel in der Einführposition befindet, durch das erste Loch und das zweite Loch erstreckt und die Arme nach innen vorgespannt sind, wobei als Reaktion darauf, dass die Einführnadel proximal aus dem ersten Loch und dem zweiten Loch herausgezogen wird, der erste Arm und der zweite Arm sich elastisch nach außen bewegen, so dass sich das erste Loch und das zweite Loch voneinander weg bewegen und die Klemme die Öffnung blockiert.

7. Kathetersystem nach Anspruch 6, bei welchem der erste Arm und der zweite Arm an einer ersten Biegung (96) verbunden sind.

8. Kathetersystem nach Anspruch 7, bei welchem der erste Arm eine zweite Biegung (98) und der zweite Arm eine dritte Biegung (100) aufweist, wobei das erste Loch nach innen zur zweiten Biegung angeordnet ist, wobei das zweite Loch nach innen zur dritten Biegung angeordnet ist.

9. Kathetersystem nach Anspruch 8, bei welchem der erste Arm ein erstes Ende (102) umfasst, wobei der zweite Arm ein zweites Ende (104) umfasst, wobei als Reaktion darauf, dass die Einführnadel proximal aus dem ersten Loch und dem zweiten Loch herausgezogen wird, das erste Ende und das zweite Ende einander überlappen.

## Revendications

1. Système de cathéter (30), comprenant :
un ensemble cathéter (32), comprenant :
un adaptateur de cathéter (34), comprenant une extrémité distale (76), une extrémité proximale (44) et une lumière (80) s'étendant à travers l'extrémité distale et l'extrémité proximale; et
un cathéter (36) s'étendant de manière distale à partir de l'extrémité distale de l'adaptateur de cathéter ; et
un ensemble aiguille (38), comprenant :
un embout d'aiguille (40) proche et proximal par rapport à l'adaptateur de cathéter ;
une aiguille d'introduction (42) s'étendant de manière distale à partir de l'embout d'aiguille ;
une canule (54) s'étendant de manière proximale à partir de l'embout d'aiguille ; et
**CARACTÉRISÉ EN CE QUE**
une gaine élastomère (56) est couplée à l'embout d'aiguille, dans lequel une extrémité proximale de la canule est enveloppée dans la gaine élastomère, dans lequel la gaine élastomère comprend une extrémité distale ouverte (72) et une extrémité proximale fermée (74),
l'ensemble cathéter comprend en outre un clip (86) disposé de manière distale par rapport à une ouverture (84) de l'extrémité proximale de l'adaptateur de cathéter, dans lequel le clip comprend un premier trou (90), dans lequel l'aiguille d'introduction s'étend à travers le premier trou lorsque l'aiguille d'introduction est dans une position d'insertion.

2. Système de cathéter de la revendication 1, dans lequel le cathéter comprend une encoche de retour (85).

3. Système de cathéter de la revendication 1, dans lequel la canule est configurée pour s'insérer dans un tube de prélèvement sanguin (60), dans lequel, en réponse au fait que le tube de prélèvement sanguin pousse la gaine élastomère de manière distale, la canule perce la gaine élastomère.

4. Système de cathéter de la revendication 1, dans lequel l'adaptateur de cathéter comprend un orifice latéral (63), dans lequel le système de cathéter comprend en outre un autre tube d'extension (65), dans lequel l'autre tube d'extension comprend une extrémité distale et une extrémité proximale, dans lequel l'extrémité distale de l'autre tube d'extension est intégrée à l'orifice latéral, dans lequel l'extrémité proximale de l'autre tube d'extension est intégrée à un adaptateur (67).

5. Système de cathéter de la revendication 1, comprenant en outre un protecteur d'aiguille (64) au moins partiellement disposé à l'intérieur de l'adaptateur de cathéter, dans lequel, en réponse au retrait de l'aiguille d'introduction de l'adaptateur de cathéter, le protecteur d'aiguille recouvre une pointe distale tranchante (66) de l'aiguille d'introduction.

6. Système de cathéter de la revendication 1, dans lequel le clip comprend un premier bras (88) présentant le premier trou (90) et un second bras (92) présentant un second trou (94), dans lequel, en réponse au fait que l'aiguille d'introduction se trouve dans la position d'insertion, l'aiguille d'introduction s'étend à travers le premier trou et le second trou, et les bras sont sollicités vers l'intérieur, dans lequel, en réponse au fait que l'aiguille d'introduction est retirée de manière proximale du premier trou et du second trou, le premier bras et le second bras se déplacent de manière élastique vers l'extérieur, de sorte que le premier trou et le second trou s'éloignent l'un de l'autre et que le clip bloque l'ouverture.

7. Système de cathéter de la revendication 6, dans lequel le premier bras et le second bras sont reliés au niveau d'un premier coude (96).

8. Système de cathéter de la revendication 7, dans lequel le premier bras comprend un deuxième coude (98) et le second bras comprend un troisième coude (100), dans lequel le premier trou est disposé vers l'intérieur par rapport au deuxième coude, dans lequel le second trou est disposé vers l'intérieur par rapport au troisième coude.

9. Système de cathéter de la revendication 8, dans lequel le premier bras comprend une première extrémité (102), dans lequel le second bras comprend une seconde extrémité (104), dans lequel, en réponse au fait que l'aiguille d'introduction est retirée de manière proximale du premier trou et du second trou, la première extrémité et la seconde extrémité se chevauchent.
